# EUROPEAN PATENT APPLICATION

(11) **EP 3 263 053 A1**
(43) Date of publication of application: **03.01.2018**
(21) Application number: 16755422.9
(22) Date of filing: 22.02.2016
(51) Int. Cl.: A61B 17/28, B25J 15/08

(54) **MEDICAL TREATMENT INSTRUMENT**

(30) Priority: 26.02.2015 JP 2015036931
(71) Applicant: Olympus Corporation, Hachioji-shi, Tokyo 192-8507 (JP)
(72) Inventor: HASEGAWA, Mitsuaki, Hachioji-shi, Tokyo 192-8507 (JP); YOSHII, Toshihiro, Hachioji-shi, Tokyo 192-8507 (JP)
(74) Representative: Gunzelmann, Rainer
(86) International application number: PCT/JP2016/055089
(87) International publication number: WO 2016/136676

(57) **Abstract**

A medical treatment instrument of the present invention includes: a pair of opening and closing pieces; an opening and closing shaft section configured to hold the pair of opening and closing pieces so that the pair of opening and closing pieces are able to pivot relative to each other; a first gear fixed to either of the pair of opening and closing pieces to pivot about the opening and closing shaft section; a second gear which is able to rotate to transmit a rotational force to the first gear; a rotatable member fixed to the second gear coaxially with the second gear and having a diameter larger than a diameter of the second gear; and a power transmission member provided around an outer circumference of the rotatable member and configured to rotate the rotatable member.

## Description

### [Technical Field]

The present invention relates to a medical treatment instrument.

Priority is claimed on Japanese Patent Application No. 2015-036931, filed February 26, 2015, the content of which is incorporated herein by reference.

### [Background Art]

In the related art, a medical treatment instrument configured to grasp or dissect a treatment target region is known.

For example, Patent Document 1 discloses a medical opening/closing forceps in which a pair of opening and closing pieces can be operated using a power transmission mechanism. To be specific, the power transmission mechanism is configured to move a first pulling wire and a second pulling wire so that, when any one of the first pulling wire and the second pulling wire is pulled, the other of the first pulling wire and the second pulling wire moves forward. In addition, the medical opening/closing forceps is configured such that the pair of opening and closing pieces can be operated using a pulling force with respect to the first pulling wire or the second pulling wire.

In the medical opening/closing forceps disclosed in Patent Document 1, the first pulling wire or the second pulling wire does not need to be pushed to move the first pulling wire or the second pulling wire forward. Thus, the wires are not easily folded.

### [Citation List]

### [Patent Literature]

### [Patent Document 1]

Japanese Patent No. 5500715

### [Summary of Invention]

### [Technical Problem]

In the medical opening/closing forceps disclosed in Patent Document 1, in order to apply a great opening and closing driving force to the opening and closing pieces, the first pulling wire or the second pulling wire needs to be pulled by a strong force. In this case, an excessive load is likely to be applied to the first pulling wire or the second pulling wire or friction in insertion passages of the first pulling wire or the second pulling wire is likely to increase. For this reason, there is a limit on a force which can be applied to the opening and closing pieces in the technology disclosed in Patent Document 1.

The present invention was made in view of the above-described circumstances, and an object of the present invention is to provide a medical treatment instrument capable of applying a great opening and closing driving force to opening and closing pieces.

### [Solution to Problem]

A medical treatment instrument related to a first aspect of the present invention includes:
a pair of opening and closing pieces; an opening and closing shaft section configured to hold the pair of opening and closing pieces so that the pair of opening and closing pieces are able to pivot relative to each other; a first gear fixed to one of the pair of opening and closing pieces to pivot about the opening and closing shaft section; a second gear which is able to rotate to transmit a rotational force to the first gear; a rotatable member fixed to the second gear coaxially with the second gear and having a diameter larger than a diameter of the second gear; and a power transmission member provided around an outer circumference of the rotatable member and configured to rotate the rotatable member.

According to a second aspect of the present invention, in the medical treatment instrument, a diameter of the first gear may be larger than the diameter of the second gear.

According to a third aspect of the present invention, the medical treatment instrument may further include a hollow housing configured to surround the opening and closing shaft section, the first gear, the second gear, and the rotatable member.

According to a fourth aspect of the present invention, in the medical treatment instrument, the first gear may be configured to be located inside the housing at all times in a process in which the opening and closing pieces pivot from a maximum closing angle to a maximum opening angle.

According to a fifth aspect of the present invention, in the medical treatment instrument, the rotatable member may have a pair of rotating plate members configured to place the second gear between the pair of rotating plate members, separated from each other, and having disc shapes with the same diameter, and the power transmission member may include a first wire wound around a first rotating plate member of the pair of rotating plate members in a predetermined first direction in directions of rotation of the rotatable member and a second wire wound around a second rotating plate member of the pair of rotating plate members in a second direction opposite to the first direction in the directions of rotation of the rotatable member.

According to a sixth aspect of the present invention, in the medical treatment instrument, a rotating shaft of the second gear may be parallel to a rotating shaft of the first gear.

According to a seventh aspect of the present invention, in the medical treatment instrument, the first gear may be fixed to a first opening and closing piece of the pair of opening and closing pieces, a second opening and closing piece of the pair of opening and closing pieces may have a third gear with a rotating shaft parallel to a rotating shaft of the first gear, and the second gear may transmit a rotational force to the first gear and the third gear such that the first gear and the third gear rotate in opposite directions.

According to an eighth aspect of the present invention, in the medical treatment instrument, the first gear may be fixed to the first opening and closing piece of the pair of opening and closing pieces, and the second opening and closing piece of the pair of opening and closing pieces may be coupled to the first opening and closing piece via a link such that the pair of opening and closing pieces pivot about the opening and closing shaft section in opposite directions.

### [Advantageous Effects of Invention]

According to a medical treatment instrument related to the aspects, a medical treatment instrument capable of applying a great opening and closing driving force to opening and closing pieces can be provided.

### [Brief Description of Drawings]

Fig. 1 is an overall diagram of a medical manipulator system including a medical treatment instrument related to a first embodiment of the present invention.
Fig. 2 is a schematic diagram showing a portion of a surgical instrument of the medical manipulator system of the first embodiment of the present invention.
Fig. 3 is a partial cross-sectional view of a medical treatment instrument serving as a portion of the surgical instrument of the first embodiment of the present invention.
Fig. 4 is a schematic plan view showing an internal structure of a treatment section of the medical treatment instrument of the first embodiment of the present invention using a partial cross section.
Fig. 5 is a schematic side view showing the internal structure of the treatment section of the first embodiment of the present invention using a partial cross section.
Fig. 6 is a schematic diagram showing an action of the medical treatment instrument of the first embodiment of the present invention.
Fig. 7 is a schematic diagram showing an action of the medical treatment instrument of the first embodiment of the present invention.
Fig. 8 is a schematic diagram showing a constitution of a modified example of the first embodiment of the present invention.
Fig. 9 is a schematic side view showing a treatment section of a medical treatment instrument related to a second embodiment of the present invention.
Fig. 10 is a perspective view showing a portion of the treatment section of the medical treatment instrument related to the second embodiment of the present invention.
Fig. 11 is a schematic side view showing another constitution example of the treatment section of the second embodiment of the present invention.
Fig. 12 is a schematic side view showing a constitution of a treatment section of a modified example of the second embodiment of the present invention.
Fig. 13 is a schematic plan view showing the constitution of the treatment section of the modified example of the second embodiment of the present invention.
Fig. 14 is a view showing an action of the treatment section of the modified example of the second embodiment of the present invention.
Fig. 15 is a view showing an action of the treatment section of the modified example of the second embodiment of the present invention.
Fig. 16 is a view showing an action of the treatment section of the modified example of the second embodiment of the present invention.

### [Description of Embodiments]

### (First embodiment)

A first embodiment of the present invention will be described using a medical manipulator system including a medical treatment instrument related to this embodiment as an example.

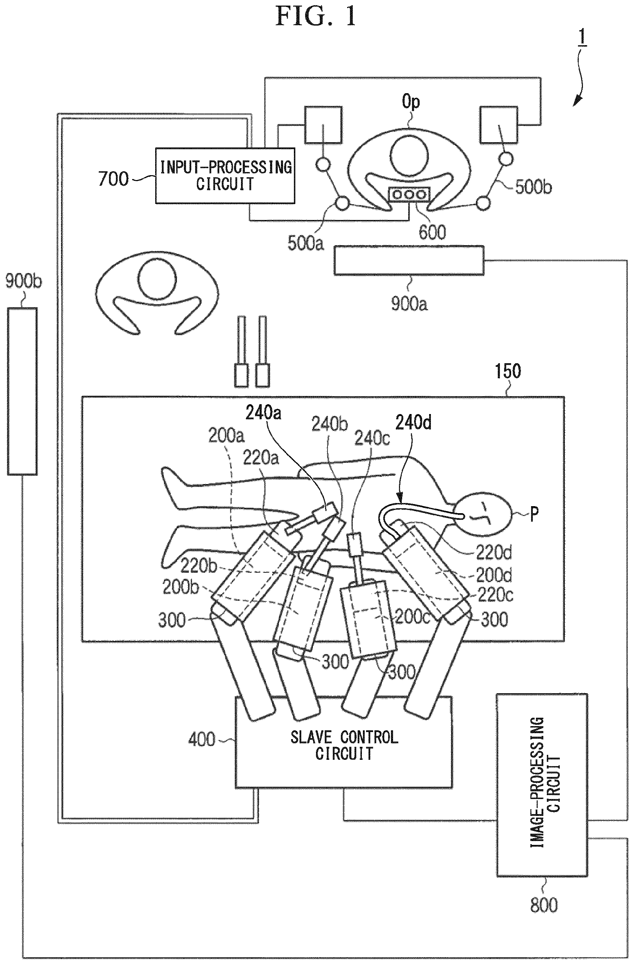

First, a schematic constitution of the entire medical manipulator system of this embodiment will be described. Fig. 1 is an overall diagram of a medical manipulator system 1 in the first embodiment of the present invention.

The medical manipulator system 1 in this embodiment shown in Fig. 1 is a master and slave type of operator system. The master and slave type of medical manipulator system 1 is a system which has two types of arm including master arms and slave arms and causes the slave arms to follow an operation of the master arms to remotely control the slave arms.

The medical manipulator system 1 shown in Fig. 1 has an surgical table 150, slave arms 200a, 200b, 200c, and 200d, a slave control circuit 400, master arms 500a and 500b, an operating section 600, an input-processing circuit 700, an image-processing circuit 800, an operator display 900a, and an assistant display 900b.

Hereinafter, in order to simplify the description, reference numerals in alphabetical order "Xa, Xb, ..., Xz" are represented as "Xa to Xz" in some cases. For example, the expression "slave arms 200a, 200b, 200c, and 200d" is represented as the expression "slave arms 200a to 200d" in some cases.

The surgical table 150 is a table on which a patient P serving as a target to be observed or treated is placed. The plurality of slave arms 200a to 200d are installed near the surgical table 150. Note that the slave arms 200a to 200d may be installed at the surgical table 150.

The slave arms 200a to 200d are configured to have a plurality of multiple-degrees-of-freedom joints. Also, the slave arms 200a to 200d have surgical instruments 240a to 240d which can be attached to and detached from the slave arms 200a to 200d.

The multiple-degrees-of-freedom joints of the slave arms 200a to 200d position the surgical instruments 240a to 240d or the like mounted at distal end sides (which are defined as a side which is directed to a body cavity of the patient P) of the slave arms 200a to 200d with respect to the patient P placed on the surgical table 150 using a bending operation.

The multiple-degrees-of-freedom joints are individually driven by a driving unit (not shown). An operation of a driving unit, which can use a motor having a servomechanism including, for example, an incremental encoder, a decelerator, and the like, as the driving unit is controlled by the slave control circuit 400.

In Fig. 1, reference numerals 220a, 220b, 220c, and 220d are surgical power transmission adapters. The surgical power transmission adapters 220a, 220b, 220c, and 220d are adapters which are disposed between the slave arms 200a, 200b, 200c, and 200d and surgical instruments 240a, 240b, 240c, and 240d and couple the slave arms 200a, 200b, 200c, and 200d and the surgical instruments 240a, 240b, 240c, and 240d.

The surgical instruments 240a to 240d may be rigid or flexible. In other words, as the surgical instruments 240a to 240d, surgical instruments configured to operate effectors such as treatment sections configured to treat a living body and joint sections by pushing or pulling rigid rods and surgical instruments configured to operate effectors operated in a living body to treat the body by pulling flexible wires can be appropriately selected and adopted. Note that, even when the surgical instruments 240a to 240d are rigid, the surgical instruments 240a to 240d may operate effectors by pulling flexible wires.

Fig. 1 shows an example in which the surgical instruments 240a to 240c are rigid and the surgical instrument 240d is flexible. The flexible surgical instrument 240d is introduced into a body via the digestive tract and the like from a natural orifice of a patient such as, for example, the mouth.

The slave control circuit 400 has, for example, a central processing unit (CPU), a memory, and the like. The slave control circuit 400 stores a predetermined program used to control the slave arms 200a to 200d, and controls operations of the slave arms 200a to 200d or the surgical instruments 240a to 240d in accordance with a control signal from the input-processing circuit 700. In other words, the slave control circuit 400 specifies a slave arm serving as one of operation objects of master arms operated by an operator Op on the basis of the control signal from the input-processing circuit 700, and calculates an amount of driving which is necessary to move the specified slave arm or the like in accordance with an amount of operation of one of the master arms of the operator Op.

The slave control circuit 400 controls an operation of the slave arm or the like serving as the operation object of the master arm in accordance with the calculated amount of driving. At this time, the slave control circuit 400 inputs a driving signal to a corresponding slave arm, and controls a magnitude or a polarity of the driving signal such that the amount of driving of the slave arm serving as the operation object has a target amount of driving in accordance with a detected signal input from a position detector of the driving unit in accordance with an operation of the corresponding slave arm.

The master arms 500a and 500b are constituted of a plurality of link mechanisms. Position detectors such as, for example, an incremental encoder are provided at links constituting the link mechanisms. The position detectors detect operations of the links so that amounts of operation of the master arms 500a and 500b are detected in the input-processing circuit 700.

In the medical manipulator system 1 shown in Fig. 1, two arms, i.e., the master arms 500a and 500b are used to operate four slave arms, and the slave arm serving as the operation object of the master arm needs to be appropriately switched. Such switching is performed, for example, by an operation of the operating section 600 of the operator Op. Of course, such switching is not required as long as the number of master arms and the number of slave arms are the same, and the master arms are in 1-to-1 correspondence with operation objects.

The operating section 600 has various operating members such as a switching button, a scaling changing switch, and a foot switch. The switching button is provided to switch the slave arm serving as the operation object of the master arms 500a and 500b. The scaling changing switch is a switch configured to change an operation ratio between maters and slaves. The foot switch is provided for emergency stopping the system. When the operator Op operates any of the operating members constituting the operating section 600, an operation signal according to an operation of the corresponding operating member is input from the operating section 600 to the input-processing circuit 700.

The input-processing circuit 700 analyzes operation signals from the master arms 500a and 500b and an operation signal from the operating section 600, generates a control signal used to control this medical manipulator system 1 in accordance with analysis results of the operation signals, and inputs a control signal to the slave control circuit 400.

The image-processing circuit 800 performs various types of image processing for displaying an image signal input from the slave control circuit 400, and generates image data used when an image is displayed on the operator display 900a or the assistant display 900b. The operator display 900a and the assistant display 900b display an image based on image data generated in the image-processing circuit 800 in accordance with an image signal acquired through an observing instrument. The operator display 900a and the assistant display 900b are constituted of, for example, liquid crystal displays.

In the medical manipulator system 1 constituted in this way, if the operator Op operates the master arms 500a and 500b, the corresponding slave arm and the surgical instruments 240a to 240d attached to the slave arm are operated in response to a motion of the master arms 500a and 500b. Thus, a desired procedure can be performed on the patient P.

Reference numeral 300 is a drape used to divide the medical manipulator system 1 into a site (a clean area) on which sterilization treatment is performed and a site (an unclean area) on which sterilization treatment is not performed.

Next, an example of a constitution of the surgical instruments 240a to 240d in this embodiment will be described.

The surgical instruments 240a to 240d include a medical instrument having a function of observing an inside of a body as it is known as a so-called endoscope or laparoscope and a medical instrument having a function of grasping or dissecting a treatment target region in a body. Hereinafter, an exemplary example of the constitution of the flexible surgical instrument 240d introduced into the digestive tract of a patient via a natural orifice of the patient such as the mouth will be described.

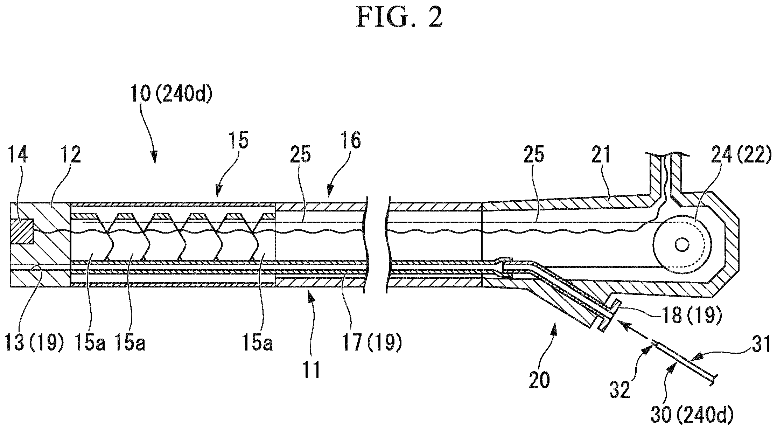

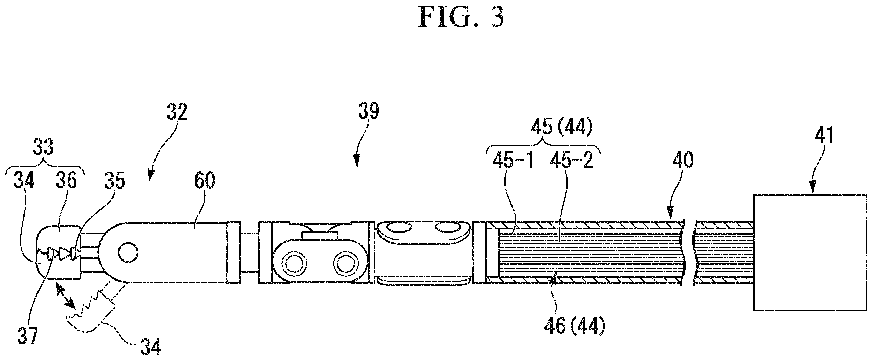

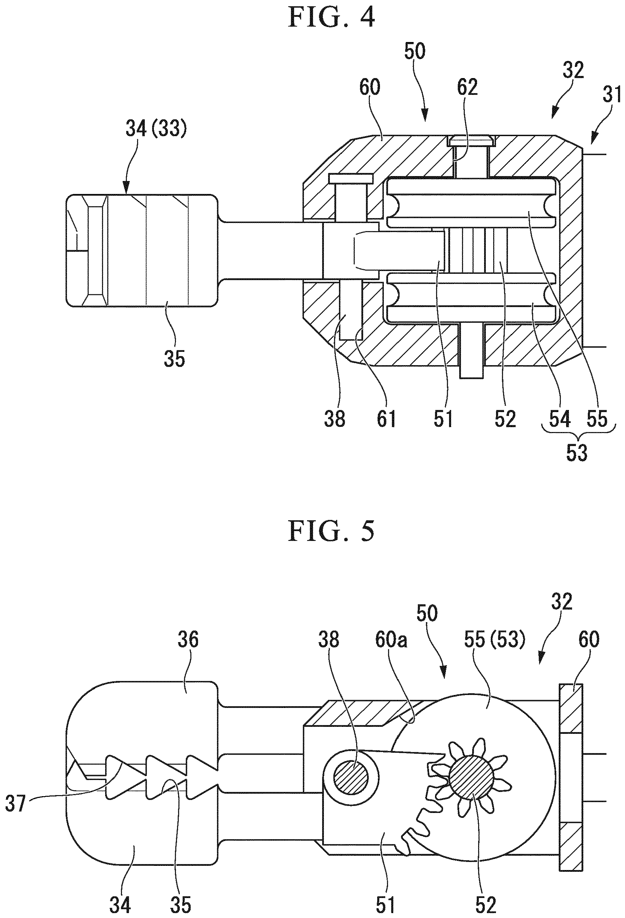

Fig. 2 is a schematic diagram showing a portion of the surgical instrument 240d of the medical manipulator system 1. Fig. 3 is a partial cross-sectional view of a medical treatment instrument 30 serving as a portion of the surgical instrument 240d. Fig. 4 is a schematic plan view showing an internal structure of a treatment section 32 of the medical treatment instrument 30 using a partial cross section. Fig. 5 is a schematic side view showing the internal structure of a treatment section 32 using a partial cross section.

As shown in Figs. 2 and 3, the surgical instrument 240d includes a guide instrument 10 which can be inserted into the body and the medical treatment instrument 30 (hereinafter simply referred to as a "treatment instrument") attached to the guide instrument 10. Both of the guide instrument 10 and the treatment instrument 30 in this embodiment are attached to the surgical power transmission adapter 220d shown in Fig. 1. Both of the guide instrument 10 and the treatment instrument 30 can be attached to or detached from the surgical power transmission adapter 220d as necessary.

The guide instrument 10 shown in Fig. 2 is an instrument which is inserted into the body to guide the treatment instrument 30 up to the treatment target region in the body. The guide instrument 10 includes a long insertion section 11 inserted into the body and an operating section 20 arranged at a proximal end section of the insertion section 11.

The guide instrument 10 is a so-called endoscope which can observe an inside of a body. Note that it is not necessary for the guide instrument 10 to be able to observe the inside of the body.

As shown in Fig. 2, the insertion section 11 includes a rigid distal end section 12, an imaging section 14, an actively bending section 15, and a flexible tube section 16.

The rigid distal end section 12 has a channel conduit line 13 through which the treatment instrument 30 is inserted. The imaging section 14 which will be described later is arranged at the rigid distal end section 12.

The channel conduit line 13 is a linear conduit line. A treatment section 32 and a joint section 39 of the treatment instrument 30, which will be described later, can be inserted through the channel conduit line 13. Dimensions of the channel conduit line 13 are not particularly limited as long as the channel conduit line 13 can be inserted through a treatment section 32 and the joint section 39. For example, an inner diameter of the channel conduit line 13 may be set such that the channel conduit line 13 can hold a treatment section 32 and the joint section 39 in a substantially linear shape. The length of the channel conduit line 13 along a central line of the channel conduit line 13 may have a length in which a treatment section 32 and the joint section 39 of the treatment instrument 30 can be accommodated in the channel conduit line 13.

The imaging section 14 includes, for example, an optical system and an image sensor (which are not shown in the drawings) so that an outside of the insertion section 11 can be captured. A constitution of the imaging section 14 is not particularly limited.

The actively bending section 15 is a tubular section arranged between the rigid distal end section 12 and the flexible tube section 16. The actively bending section 15 has, for example, a plurality of tubular joint rings 15a which are arranged and bendably coupled to each other in a longitudinal axis direction of the insertion section 11. In this case, an angle wire 25 which will be described later is coupled to a joint ring among the plurality of joint rings which is at a distal end side. The actively bending section 15 can be deformed in a curved shape by being pulled by the angle wire 25 in response to an operation at the operating section 20.

The flexible tube section 16 is a flexible tubular member. Signal lines (not shown) coupled to the imaging section 14, the angle wire 25 which will be described later, and the like are arranged inside the flexible tube section 16. Also, the flexible tube section 16 has a channel tube 17 and an opening member 18 configured to introduce the treatment instrument. A distal end of the channel tube 17 is coupled to a proximal end of the channel conduit line 13 of the rigid distal end section 12. The opening member 18 is coupled to a proximal end of the channel tube 17.

The channel tube 17 is a tube into which an insert 31 of the treatment instrument 30 can be inserted. The channel tube 17 can be flexibly deformed in accordance with deformation of the flexible tube section 16. The distal end of the channel tube 17 communicates with the proximal end of the channel conduit line 13. The proximal end of the channel tube 17 communicates with a distal end of the opening member 18.

The opening member 18 is arranged at the operating section 20. The opening member 18 is constituted of a rigid or flexible tubular member into which the insert 31 (refer to Fig. 3) of the treatment instrument 30 is inserted. The opening member 18 is located outside of the body at a time of using the treatment instrument 30.

In this embodiment, as shown in Fig. 2, a treatment instrument channel 19 is constituted of the channel conduit line 13 which is provided at the rigid distal end section 12 and the channel tube 17 and the opening member 18 which are provided in the flexible tube section 16. The insert 31 of the treatment instrument 30 is inserted into the treatment instrument channel 19. In this embodiment, the guide instrument 10 may be adopted as long as the guide instrument 10 has the treatment instrument channel 19, and other constitution elements are not necessarily required.

The operating section 20 has a casing 21 and an angle knob 22. The casing 21 is arranged at a proximal end of the flexible tube section 16. The angle knob 22 is attached to the casing 21.

The angle knob 22 includes a sprocket or a pulley 24 (hereinafter referred to as a "sprocket 24 or the like") arranged inside the casing 21. The angle knob 22 is constituted to be able to be operated using the master arms 500a and 500b shown in Fig. 1 by a driving device (not shown).

The angle wire 25 is coupled to the sprocket 24 or the like of the angle knob 22. For this reason, the angle wire 25 is operated through the sprocket 24 or the like by rotating the angle knob 22 so that the actively bending section 15 can be bent.

The angle wire 25 is coupled to the actively bending section 15 and the angle knob 22, and an amount of force of a rotating operation with respect to the angle knob 22 is transmitted to the actively bending section 15. In this embodiment, a plurality of angle wires 25 are provided corresponding to directions the actively bending section 15 can bend. The plurality of angle wires 25 can be independently operated by the angle knob 22.

Next, a constitution of the treatment instrument 30 inserted into and used in the treatment instrument channel 19 of the guide instrument 10 of this embodiment will be described.

The treatment instrument 30 shown in Fig. 3 includes the insert 31 and a detachable section 41. The insert 31 is inserted into the treatment instrument channel 19 of the guide instrument 10 shown in Fig. 2, and is configured to be able to project from or retract to a distal end of the insertion section 11. The detachable section 41 is arranged at a proximal end of the insert 31.

As shown in Fig. 3, the insert 31 includes a treatment section 32, the joint section 39, a flexible section 40, and a transmission member 44.

A treatment section 32 shown in Figs. 3 and 4 is provided at a distal end of the insert 31 to treat the treatment target region in the body. A constitution which has a member configured to be opened or closed to perform the treatment is adopted for a treatment section 32. A well-known constitution configured to perform grasping, dissection, cauterization, suturing, observation, or other treatment may be appropriately selected and adopted for a treatment section 32. Examples of a treatment section 32 can include forceps, scissors, marking devices, suturing devices, and the like. Hereinafter, as an exemplary example of a treatment section 32, a constitution in which tissue or the like at a treatment target region can be grasped is described.

As shown in Figs. 3 to 5, a treatment section 32 in this embodiment includes a pair of jaws 33 (a pair of opening and closing pieces), an opening and closing shaft section 38, a deceleration mechanism 50, and a housing 60. The pair of jaws 33 are constituted of a first jaw 34 and a second jaw 36. The opening and closing shaft section 38 supports the first jaw 34 to pivot relative to the second jaw 36. The deceleration mechanism 50 is provided to transmit an opening and closing driving force to the pair of jaws 33. The housing 60 is a hollow member configured to surround the deceleration mechanism 50.

The first jaw 34 of the pair of jaws 33 is a first opening and closing piece having a first grasping surface 35 configured to grasp the treatment target region.

The first jaw 34 can be opened or closed by pivoting about a central line of the opening and closing shaft section 38. The opening and closing shaft section 38 is provided to be integrally molded with the first jaw 34 in this embodiment. It is not necessary for the first jaw 34 and the opening and closing shaft section 38 to be integrally molded. The first jaw 34 is opened or closed by a force transmitted from the detachable section 41 which will be described later to the first jaw 34 via a wire (a first transmission member 45 which will be described later) and the deceleration mechanism 50.

The second jaw 36 of the pair of jaws 33 is a second opening and closing piece having a second grasping surface 37 configured to grasp the treatment target region. The second jaw 36 is fixed to the housing 60 which will be described later.

The pair of jaws 33 can grasp tissue in a state where the tissue is sandwiched between the first grasping surface 35 and the second grasping surface 37 by opening or closing the first jaw 34 with respect to the second jaw 36 as shown in Fig. 3. Also, a movable range of the pair of jaws 33 is defined in a predetermined range such that the movable range is between a maximum closed angle in which the first grasping surface 35 and the second grasping surface 37 are closest to and face each other and a maximum opening angle. The maximum opening angle is an angle at which the pair of jaws 33 have been moved such that the pair of jaws 33 have been opened from the maximum closed angle using the opening and closing shaft section 38 as a center such that the first grasping surface 35 and the second grasping surface 37 are the farthest away from each other. In this embodiment, the maximum closed angle of the pair of jaws 33 is an angle at which the first grasping surface 35 comes in contact with the second grasping surface 37. In this embodiment, the maximum opening angle of the pair of jaws 33 is an angle at which a first gear 51 comes into contact with a stopper 60a formed in the housing 60 to be able to come into contact with a portion of the first gear 51, which will be described later, fixed to the first jaw 34.

As shown in Figs. 4 and 5, the deceleration mechanism 50 includes the first gear 51, a second gear 52, and a pulley 53. The first gear 51 is fixed to the first jaw 34. The second gear 52 can be pivoted to transmit a rotational force to the first gear 51. The pulley 53 is fixed to the second gear 52 coaxially with the second gear 52.

The first gear 51 is a gear in which teeth meshed with the second gear 52 are formed on an outer circumference of a circular disk which is coaxial with the opening and closing shaft section 38 of the first jaw 34. The first gear 51 is formed as a portion of a gear of which a diameter is larger than that of the second gear 52, and which has a fan shape. An arc portion of the first gear 51, which has a fan shape, may be set to a length of about 1/6 to 1/4 of a circumference thereof to correspond to a movable range of the first jaw 34. The first gear 51 is preferably configured such that the first gear 51 does not protrude from a treatment section 32. For example, in this embodiment, the first gear 51 is preferably accommodated only inside the housing 60. The first gear 51 has more preferably a large diameter which is within a range in which the first gear 51 does not protrude from a treatment section 32.

A movable range of the first gear 51 is limited by the stopper 60a formed in the housing 60 such that the first gear 51 is located inside the housing 60 when the pair of jaws 33 have the maximum opening angle. With this constitution, in this embodiment, the first gear 51 is located inside the housing 60 at all times in a process in which the pair of jaws 33 are opened or closed between the maximum closed angle and the maximum opening angle. As a result, since the first gear 51 does not serve as a protrusion to define a maximum outer diameter of a treatment section 32, the first gear 51 does not interfere with living body tissue, an endoscope, and the like. The first gear 51 can be formed in a fan shape to have a diameter which is larger than an outer diameter of the housing 60 as long as the first gear 51 is within the range in which the first gear 51 does not protrude from a treatment section 32.

The first gear 51 is fixed to the first jaw 34. In this embodiment, the first gear 51 may be integrally molded with the first jaw 34.

The second gear 52 is a rotatable gear having a rotating shaft which is parallel to a rotating shaft of the first gear 51. The diameter of the second gear 52 is smaller than a diameter of the first gear 51. Note that the second gear 52 may have the same diameter as the first gear 51. A relationship between the diameter of the first gear 51 and the diameter of the second gear 52 defines a speed deceleration ratio in the deceleration mechanism 50.

The pulley 53 is a rotatable member which has a pair of rotating plate members (a first rotating plate member 54 and a second rotating plate member 55) configured to place the second gear 52 between them and which is separated from each other.

The first rotating plate member 54 is a substantially disc-shaped member. A groove around which a first wire 45-1 of the first transmission member 45 which will be described later is wound is formed on an outer circumference of the first rotating plate member 54, which is coaxial with a rotating shaft of the second gear 52. The first wire 45-1 is wound around the first rotating plate member 54 in a predetermined first direction in a direction of rotation of the pulley 53. A distal end of the first wire 45-1 is fixed to an outer circumferential surface of the first rotating plate member 54. The diameter of the first rotating plate member 54 is larger than the diameter of the second gear 52.

The second rotating plate member 55 is a substantially disc-shaped member. A groove around which a second wire 45-2 of the first transmission member 45 which will be described later is wound is formed in a disc-shaped outer circumference of the second rotating plate member 55, which is coaxial with the rotating shaft of the second gear 52. The second wire 45-2 is wound around the second rotating plate member 55 in a second direction which is opposite to the above-described predetermined first direction. A distal end of second wire 45-2 is fixed to an outer circumferential surface of the second rotating plate member 55. The diameter of the second rotating plate member 55 is larger than the diameter of the second gear 52.

As described above, in this embodiment, a diameter of the pulley 53 constituted of the first rotating plate member 54 and the second rotating plate member 55 is larger than the diameter of the second gear 52. A relationship between the diameter of the second gear 52 and the diameter of a pulley 53 defines the speed deceleration ratio in the deceleration mechanism 50.

In this embodiment, a the speed deceleration ratio in the deceleration mechanism 50 is related to diameters of the first gear 51, the second gear 52, and a pulley 53 which are factors which define the speed deceleration ratio. A relationship between these diameters is set to be a relationship in which the first gear 51, the second gear 52, and a pulley 53 can be accommodated inside the housing 60 and rotation of a pulley 53 is decelerated such that a pulley 53 is rotated once when the first gear 51 is rotated less than one revolution.

A pulley 53 and the second gear 52 constitute a rotating body configured to integrally rotate using the rotating shaft of a pulley 53 and the second gear 52 as a rotational center. For example, a rotating body with a combination of a pulley 53 and the second gear 52 may be integrally formed by cutting or molding the first rotating plate member 54 and the second gear 52, and may be formed such that the second gear 52 is fitted to the second rotating plate member 55.

The diameter of the first rotating plate member 54 and the diameter of the second rotating plate member 55 in this embodiment are equal to each other. Also, a pulley 53 is rotatably supported by the housing 60 such that the second gear 52 is at a position at which the second gear 52 and a central line of the insert 31 overlap each other.

The housing 60 is a hollow member in which the first gear 51, the second gear 52, and a pulley 53 are held at least in an inside of the housing 60. The housing 60 holds the first gear 51 and the second gear 52 inside the housing 60 such that the first gear 51 and the second gear 52 do not come into contact with tissue in the body, the guide instrument 10, or the like. The housing 60 has a bearing 61 configured to hold the opening and closing shaft section 38 and a bearing 62 configured to hold the rotating body in which the second gear 52 and a pulley 53 are formed as a single body.

The diameter of the housing 60 is smaller than an inner diameter of the treatment instrument channel 19.

The joint section 39 shown in Fig. 3 is disposed between a treatment section 32 and the flexible section 40. The joint section 39 has one or more degrees of freedom. The joint section 39 is bent due to a force transmitted from the detachable section 41, which will be described later, to the joint section 39 via a wire (a second transmission member 46 which will be described later). For example, the joint section 39 receives a force from the second transmission member 46, pivots about a predetermined pivoting shaft, and thus is bent and deformed so that a treatment section 32 can swing. A plurality of predetermined pivoting shafts in the joint section 39 may be provided spaced apart from each other. When a pulling force is not applied to the second transmission member 46, the joint section 39 can be freely bent and deformed due to an external force in a range of degrees of freedom thereof. The detailed constitution of the joint section 39 is not particularly limited. Note that the joint section 39 may not be provided in the treatment instrument 30 in this embodiment.

The flexible section 40 is a tubular member with flexibility. The transmission member 44 of the first transmission member 45 configured to operate a treatment section 32, the second transmission member 46 configured to operate the joint section 39, or the like is arranged inside the flexible section 40 in this embodiment. The flexible section 40 is longer than a total length of the treatment instrument channel 19. For this reason, when a distal end of the flexible section 40 is located at a distal end of the treatment instrument channel 19, a proximal end of the flexible section 40 is located outside of the opening member 18.

The detachable section 41 is a member which can be attached to and detached from a power source (not shown) provided at the surgical power transmission adapter 220d. Power used to operate a treatment section 32 and the joint section 39 is transmitted from the above-described power source to the detachable section 41 in a state in which the detachable section 41 is attached to the above-described power source.

The transmission member 44 has the first transmission member 45 configured to open or close the pair of jaws 33 of a treatment section 32 and the second transmission member 46 configured to bend the joint section 39. In this embodiment, both of the first transmission member 45 and the second transmission member 46 are wires.

The first transmission member 45 is a power transmission member configured to transmit power used to open or close the pair of jaws 33. The first transmission member 45 has the first wire 45-1 configured to open the pair of jaws 33 and the second wire 45-2 configured to close the pair of jaws 33. Although a detailed description will be given below, in this embodiment, the pair of jaws 33 are opened when the first wire 45-1 is pulled toward a proximal end, and the pair of jaws 33 are closed when the second wire 45-2 is pulled toward the proximal end. Both of the first wire 45-1 and the second wire 45-2 constituting the first transmission member 45 can be pulled using a power source of the surgical power transmission adapters 220d (refer to Fig. 1) via the detachable section 41.

In this embodiment, the first wire 45-1 and the second wire 45-2 are configured to be relatively moved by applying a relatively strong pulling force to any of the first wire 45-1 and the second wire 45-2.

The second transmission member 46 is coupled to the joint section 39 and is coupled to the detachable section 41. The second transmission member 46 can be pulled using the power source of the surgical power transmission adapters 220d via the detachable section 41. The joint section 39 can be operated due to a pulling force applied to the second transmission member 46.

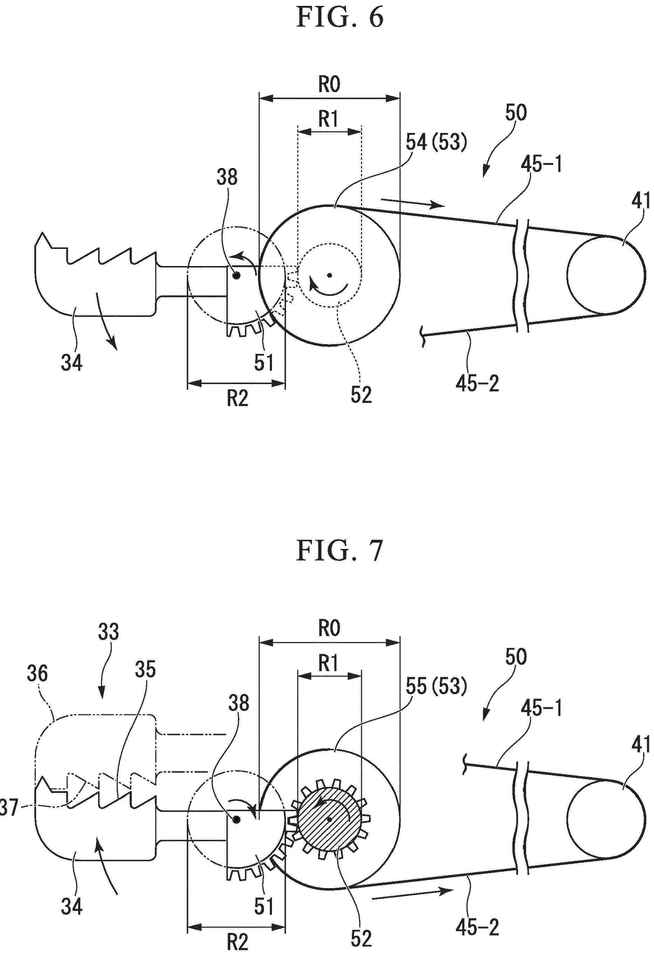

An action of the medical treatment instrument related to this embodiment will be described. Figs. 6 and 7 are schematic diagrams showing an action of the medical treatment instrument 30. As shown in Fig. 6, in this embodiment, when the first wire 45-1 is pulled toward the proximal end, the first wire 45-1 rotates the first rotating plate member 54. Since the second gear 52 is fixed to the first rotating plate member 54, the second gear 52 is rotated integrally with rotation of the first rotating plate member 54. The second gear 52 is rotated integrally with the first rotating plate member 54 so that a rotational force is transmitted to the first gear 51. Since a diameter R0 of the first rotating plate member 54 is larger than a diameter R1 of the second gear 52, a pulling force due to the first wire 45-1 is decelerated and transmitted to the first gear 51. Furthermore, in this embodiment, since a diameter R2 of the first gear 51 is larger than the diameter R1 of the second gear 52, the rotational force is decelerated as well when rotation is transmitted from the second gear 52 to the first gear 51. For this reason, in this embodiment, an opening and closing driving force transmitted to the first jaw 34 via the first gear 51 is larger than the pulling force with respect to the first wire 45-1.

As shown in Fig. 7, when the second wire 45-2 is pulled toward the proximal end, the second rotating plate member 55 is rotated by the second wire 45-2. A direction of rotation of the second rotating plate member 55 is a direction which is opposite to a direction of rotation of the first rotating plate member 54 when the first wire 45-1 is pulled. Since the second gear 52 is fixed to the second rotating plate member 55, the second gear 52 is rotate integrally with rotation of the second rotating plate member 55. The second gear 52 is rotated integrally with the second rotating plate member 55 so that a rotational force is transmitted to the first gear 51. Thus, the first gear 51 is rotated in a direction which is opposite to that when the first wire 45-1 is pulled. Since the diameter R0 of the second rotating plate member 55 is larger than the diameter R1 of the second gear 52, a pulling force due to the second wire 45-2 is decelerated and transmitted to the first gear 51. Furthermore, in this embodiment, since the diameter R2 of the first gear 51 is larger than the diameter R1 of the second gear 52, the rotational force is decelerated as well when rotation is transmitted from the second gear 52 to the first gear 51. For this reason, in this embodiment, the opening and closing driving force transmitted to the first jaw 34 via the first gear 51 is larger than the pulling force with respect to the second wire 45-2.

The second wire 45-2 is pulled toward the proximal end so that the first jaw 34 is rotated about the central line of the opening and closing shaft section 38 such that the first grasping surface 35 of the first jaw 34 approaches the second grasping surface 37 of the second jaw 36. The pair of jaws 33 are closed due to the opening and closing driving force decelerated by the deceleration mechanism 50 so that the pair of jaws 33 can be closed due to a force larger than the pulling force with respect to the second wire 45-2. As a result, a driving force can be applied to the pair of jaws 33 in a state in which a grasping target is grasped by the pair of jaws 33 without slip such as, for example, when suturing is performed while a suture needle is grasped by the pair of jaws 33.

As described above, in this embodiment, in the case of both of the opening and closing operations of the first jaw 34 with respect to the second jaw 36, the pulling force passing through the first transmission member 45 (the first wire 45-1 and the second wire 45-2) is decelerated by the deceleration mechanism 50 and is transmitted to the first jaw 34.

As described above, according to the medical treatment instrument related to this embodiment, since a treatment section 32 has the deceleration mechanism 50, a greater opening and closing driving force than the pulling force applied to the first transmission member 45 of the medical treatment instrument 30 can be given to the first jaw 34 serving as the opening and closing piece.

In addition, in this embodiment, in the case of a member which is at a side closer to the first jaw 34 than to the deceleration mechanism 50, a greater opening and closing driving force than the pulling force applied to the first transmission member 45 can be obtained. In other words, in a range from the detachable section 41 to the deceleration mechanism 50, the pulling force of the first transmission member 45 can be weaker than that when there is no the deceleration mechanism 50. The deceleration mechanism 50 is provided in a treatment section 32 so that an influence of the pulling force of the first transmission member 45 on the joint section 39 provided at a position which is closer to the detachable section 41 than to the treatment section 32 is weaker than that when there is no deceleration mechanism 50.

For example, when the deceleration mechanism 50 is not provided, it is assumed that the pulling force of the first transmission member 45 will be increased to transmit a great opening and closing driving force to a treatment section 32. In this case, the pulling force applied to the first transmission member 45 is transmitted to the joint section 39, and thus the joint section 39 is likely to be unintentionally operated (inter-joint interference occurs). On the other hand, in this embodiment, the deceleration mechanism 50 is provided in a treatment section 32 so that the influence of the pulling force applied to the first transmission member 45 is small in the case of the joint section 39. As a result, inter-joint interference due to the pulling force applied to the first transmission member 45 does not easily occur in the case of the joint section 39.

### (Modified example 1-1)

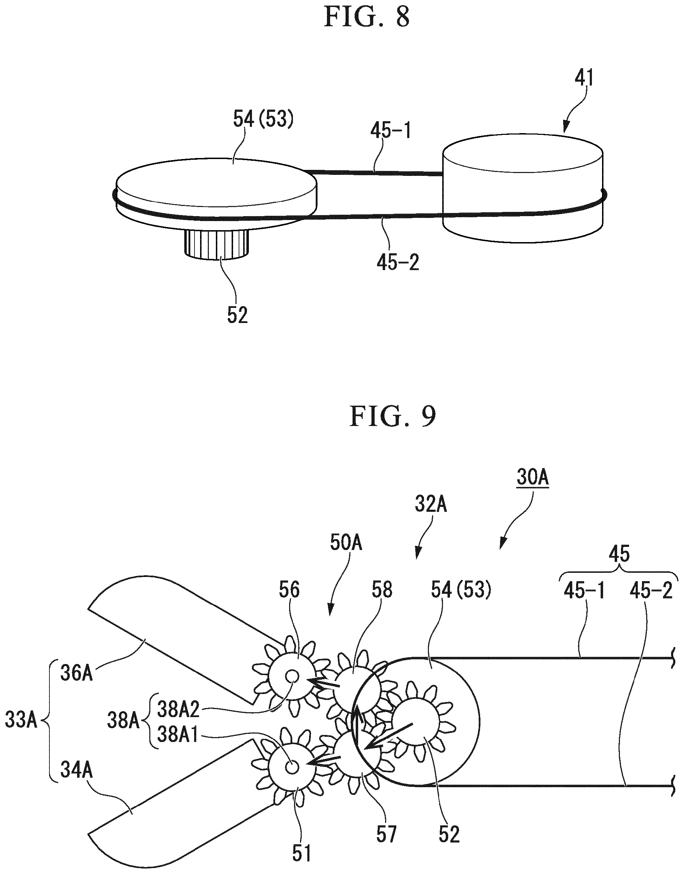

A modified example of the above-described embodiment will be described. Fig. 8 is a schematic diagram showing a constitution of Modified Example 1-1 of this embodiment.

In this modified example, as shown in Fig. 8, a pulley 53 does not have a second rotating plate member 55, and a second wire 45-2 is wound around a first rotating plate member 54 together with a first wire 45-1.

In this modified example, a distal end of the first wire 45-1 and a distal end of the second wire 45-2 may be independently fixed to the first rotating plate member 54, and the distal end of the first wire 45-1 and the distal end of the second wire 45-2 may be fixed to the first rotating plate member 54 via a coupling tube (not shown) configured to couple both the distal end of the first wire 45-1 and the distal end of the second wire 45-2.

In this modified example, a treatment section 32 can be miniaturized compared to that of the above-described first embodiment.

Note that, in this modified example, it is not necessary for a rotating shaft of a first gear 51 (refer to Fig. 5) and a rotating shaft of a second gear 52 to be strictly parallel to each other. For example, the first gear 51 and the second gear 52 may be constituted of bevel gears so that the rotating shaft of the first gear 51 is not parallel to the rotating shaft of the second gear 52.

### (Second embodiment)

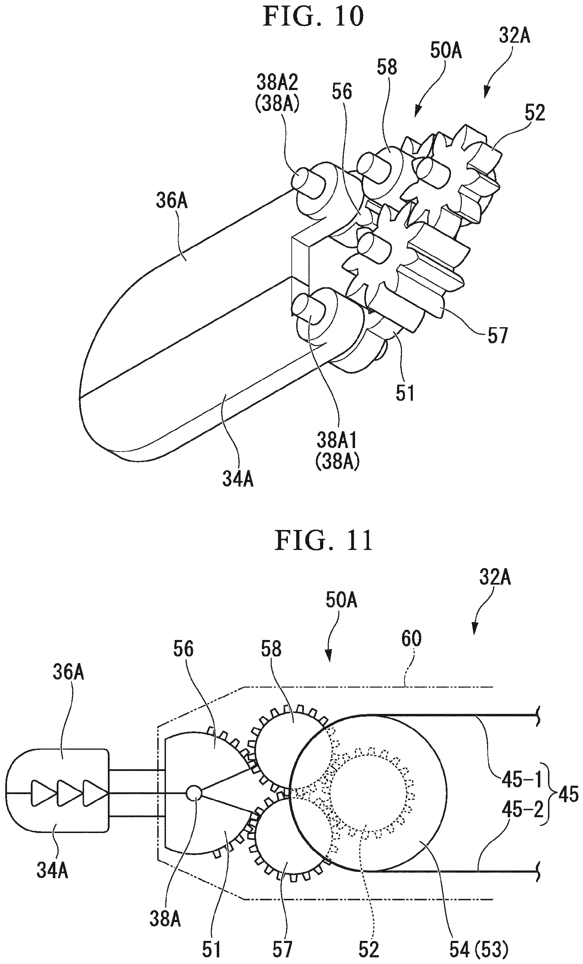

A second embodiment of the present invention will be described. Fig. 9 is a schematic side view showing a treatment section 32 of a medical treatment instrument 30A related to this embodiment. Fig. 10 is a perspective view showing a portion of the treatment section 32 of the medical treatment instrument 30A. Fig. 11 is a schematic side view showing another constitution example of a treatment section.

Note that a pulley 53 is not shown in Fig. 10.

As shown in Figs. 9 and 10, the medical treatment instrument 30A related to this embodiment includes a treatment section 32A having a different constitution from the treatment section 32 disclosed in the first embodiment.

The treatment section 32A in this embodiment includes a pair of jaws 33A and a deceleration mechanism 50A. The pair of jaws 33A have a first jaw 34A and a second jaw 36A which can pivot about a central line of an opening and closing shaft section 38A. The deceleration mechanism 50A is provided to transmit the opening and closing driving force to the pair of jaws 33A. A constitution of the pair of jaws 33A and the deceleration mechanism 50A of the treatment section 32A in this embodiment is different from that of the treatment section 32 in the above-described first embodiment.

A first opening and closing shaft section 38A1 which is the same as the opening and closing shaft section 38 disclosed in the first embodiment is formed at the first jaw 34A. In addition, a first grasping surface 35 and a first gear 51 are fixed to the first jaw 34A as in the first embodiment. In this embodiment, the first jaw 34A is integrally molded with the first opening and closing shaft section 38A1 and the first gear 51.

Unlike the above-described first embodiment, the second jaw 36A is not fixed to a housing (not shown). A second opening and closing shaft section 38A2 different from the first opening and closing shaft section 38A1 described above is formed at the second jaw 36A. A third gear 56 with the same shape and size as the first gear 51 is fixed to the second jaw 36A. In this embodiment, the second jaw 36A is integrally molded with the second opening and closing shaft section 38A2 and the third gear 56.

In this embodiment, a central line of the first opening and closing shaft section 38A1 is parallel to a central line of the second opening and closing shaft section 38A2.

As shown in Figs. 9 and 10, the third gear 56 is a gear which can rotate about a rotating shaft coaxial with a pivoting center (the central line of the second opening and closing shaft section 38A2 in this embodiment) of the second jaw 36A. The third gear 56 in this embodiment can rotate about the central line of the second opening and closing shaft section 38A2 with the second jaw 36A within a movable range of the second jaw 36A integrally.

Also, the third gear 56 in this embodiment can rotate about a rotating shaft parallel to the rotating shaft of the first gear 51.

In this embodiment, the first gear 51 is meshed with a second gear 52 via a fourth gear 57. The fourth gear 57 is a gear which can rotate about a rotating shaft parallel to the rotating shafts of the first gear 51 and the second gear 52 by receiving a rotational driving force from the second gear 52.

Furthermore, in this embodiment, the third gear 56 is meshed with the second gear 52 via the fourth gear 57 and a fifth gear 58.

The fifth gear 58 is a gear which can rotate about a rotating shaft parallel to rotating shafts of the third gear 56 and the fourth gear 57 by receiving a rotational driving force from the fourth gear 57. The fifth gear 58 rotates in a direction opposite to a direction of rotation of the fourth gear 57.

In this embodiment, when the second gear 52 rotates in one predetermined direction, the first gear 51 rotates in the same direction as that of the second gear 52, and the third gear 56 rotates in a direction opposite to that of the second gear 52. As a result, in this embodiment, the first jaw 34A and the second jaw 36A rotate in directions opposite to each other due to rotation of the second gear 52. The pair of jaws 33A in this embodiment are opened or closed using an operation in which the first jaw 34A and the second jaw 36A rotate about the opening and closing shaft section 38A in directions opposite to each other.

In this embodiment, as in Modified Example 1-1 of the first embodiment, a first wire 45-1 and a second wire 45-2 are wound around a first rotating plate member 54 of the pulley 53. Note that, as shown in the above-described first embodiment, a second rotating plate member 55 may be provided at the pulley 53 in addition to the first rotating plate member 54.

In this embodiment, the deceleration mechanism 50A is constituted of the first gear 51, the second gear 52, the third gear 56, the fourth gear 57, the fifth gear 58, and the pulley 53.

In this embodiment, unlike the above-described first embodiment, when the first wire 45-1 is pulled, the pair of jaws 33A are closed, and when the second wire 45-2 is pulled, the pair of jaws 33A are opened. Also in this embodiment, as in the first embodiment, a pulling force with respect to the first wire 45-1 and the second wire 45-2 is decelerated due to an action of the deceleration mechanism 50A with the pulley 53 and the second gear 52. In addition, in this embodiment, an opening and closing driving force decelerated by the deceleration mechanism 50A is transmitted from the second gear 52 to the first gear 51 and the third gear 56.

A treatment instrument 30A in this embodiment also accomplishes the same effects as the above-described first embodiment.

In this embodiment, since both of the first jaw 34A and the second jaw 36A are opened or closed, an allowable range of motion of the pair of jaws 33A is wide.

As shown in Fig. 11, the deceleration mechanism 50A may be configured such that the first gear 51 and the third gear 56 rotate coaxially with each other. The opening and closing shaft section 38A serving as a common rotating shaft of the first gear 51 and the third gear 56 is arranged at a substantial center in a radial direction of a housing 60 such that the opening and closing shaft section 38A crosses a central line of the housing 60. As shown in Fig. 11, when the first gear 51 is coaxial with the third gear 56, a radius of the first gear 51 and a radius of the third gear 56 can be set to be larger than when the first gear 51 is not coaxial with the third gear 56.

### (Modified example 2-1)

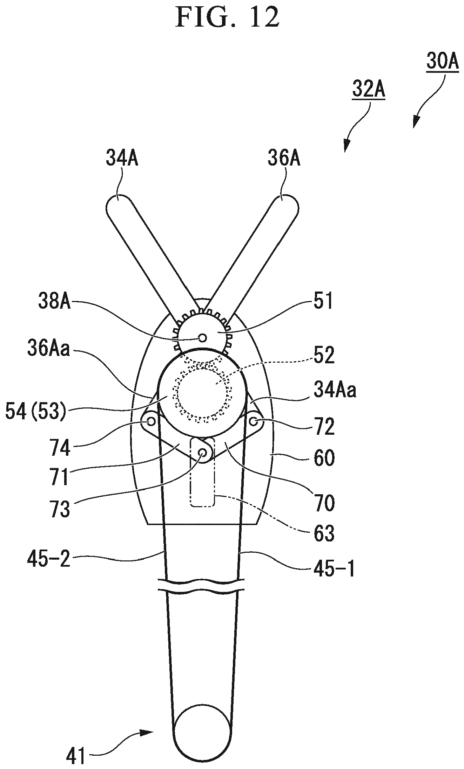

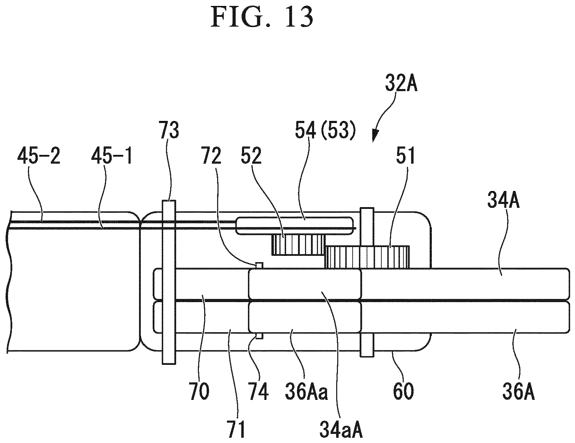

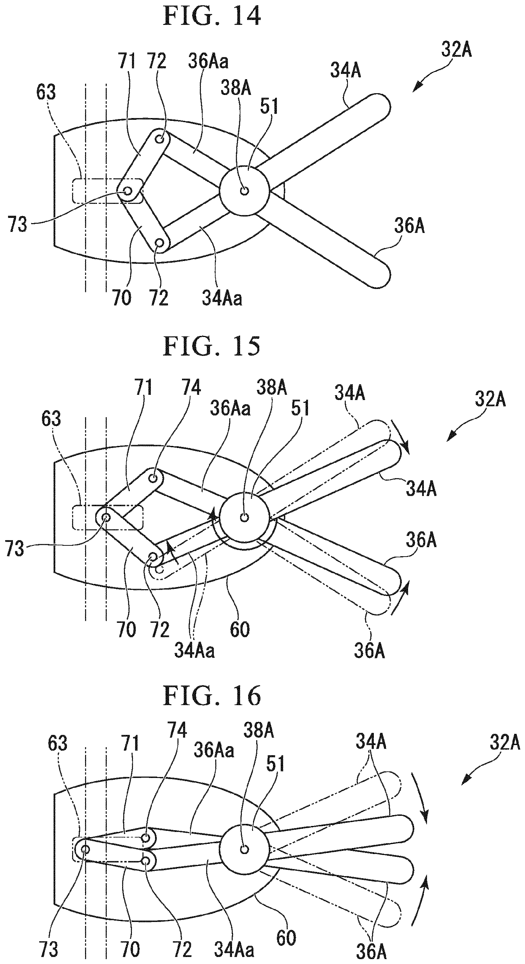

A modified example 2-1 of the second embodiment will be described. Fig. 12 is a schematic side view showing a constitution of a treatment section of this modified example. Fig. 13 is a schematic plan view showing the constitution of the treatment section of this modified example. Figs. 14 to 16 are views showing actions of the treatment section of the modified example.

In this modified example, a link 70 and a link 71 configured to couple the first jaw 34A and the second jaw 36A are provided, as shown in Figs. 12 and 13, instead of the third gear 56, the fourth gear 57, and the fifth gear 58 (refer to Figs. 9 and 10) disclosed in the above-described second embodiment.

The link 70 is a rod-like member configured to connect a proximal end section 34Aa of the first jaw 34A and the link 71 via pins 72 and 73. The pin 72 and the pin 73 are substantially cylindrical members of which central lines extend in parallel with the central line of the opening and closing shaft section 38A.

The link 71 is a rod-like member configured to connect the link 70 and a proximal end section 36Aa of the second jaw 36A via the pin 73 and a pin 74. The pin 73 and the pin 74 are substantially cylindrical members of which center lines extend in parallel with the central line of the opening and closing shaft section 38A.

A slit 63 extending in a direction which is perpendicular to the central line of the opening and closing shaft section 38A is formed in the housing 60. The pin 73 configured to couple the link 70 and the link 71 is engaged with the slit 63.

Furthermore, in this modified example, as in Modified Example 1-1 of the first embodiment, the first wire 45-1 and the second wire 45-2 are wound around the first rotating plate member 54 of the pulley 53.

Actions of the medical treatment instrument 30A in this modified example will be described using an exemplary process in which the pair of jaws 33A are closed. In the medical treatment instrument 30A of this modified example, a rotational force transmitted from the pulley 53 and the second gear 52 to the first gear 51 shown in Fig. 13, pivots the first jaw 34A such that, for example, a state shown in Fig. 14 is changed to a state shown in Fig. 15.

As shown in Figs. 15 and 16, the first jaw 34A rotates so that the proximal end section 34Aa of the first jaw 34A moves the link 70. The pin 73 configured to couple the link 70 and the link 71 is engaged with the slit 63 of the housing 60 so that the second jaw 36A pivots in a direction opposite to that of the first jaw 34A using the central line of the opening and closing shaft section 38A as the rotational center. Thus, as in the above-described second embodiment, the first jaw 34A and the second jaw 36A can be opened or closed using the central line of the opening and closing shaft section 38A as the rotational center.

Also in this modified example, as in the above-described first and second embodiments, a pulling force with respect to the first transmission member 45 (the first wire 45-1 and the second wire 45-2) is decelerated through the pulley 53 and the second gear 52 and is transmitted to the first gear 51. As a result, also in this modified example, the same effects as in the above-described first and second embodiments are accomplished.

Although embodiments of the present invention have been described above in detail with reference to the drawings, the specific constitution is not limited to the embodiments. Changes in design, etc. without departing from the gist of the present invention are also included.

In addition, constituent elements shown in the above-described embodiments and modified examples can be constituted by appropriate combination thereof.

### [Industrial Applicability]

A medical treatment instrument capable of applying a great opening and closing driving force to opening and closing pieces can be provided.

### [Reference Signs List]

30, 30A Medical treatment instrument
32, 32A Treatment section
33, 33A Pair of jaws (pair of opening and closing pieces)
34 First jaw (opening and closing piece)
36, 36A Second jaw (opening and closing pieces)
38, 38A Opening and closing shaft section
38A1 First opening and closing shaft section (opening and closing shaft section)
38A2 Second opening and closing shaft section (opening and closing shaft section)
44 Transmission member (power transmission member)
45 First transmission member (power transmission member)
45-1 First wire
45-2 Second wire
46 Second transmission member (power transmission member)
51 First gear
52 Second gear
53 Pulley (rotatable member)
54 First rotating plate member
55 Second rotating plate member
56 Third gear
60 Housing
70 Link
71 Link

## Claims

1. A medical treatment instrument comprising:
a pair of opening and closing pieces;
an opening and closing shaft section configured to hold the pair of opening and closing pieces so that the pair of opening and closing pieces are able to pivot relative to each other;
a first gear fixed to one of the pair of opening and closing pieces to pivot about the opening and closing shaft section;
a second gear which is able to rotate to transmit a rotational force to the first gear;
a rotatable member fixed to the second gear coaxially with the second gear and having a diameter larger than a diameter of the second gear; and
a power transmission member provided around an outer circumference of the rotatable member and configured to rotate the rotatable member.

2. The medical treatment instrument according to claim 1, wherein a diameter of the first gear is larger than the diameter of the second gear.

3. The medical treatment instrument according to claim 1, further comprising:
a hollow housing configured to surround the opening and closing shaft section, the first gear, the second gear, and the rotatable member.

4. The medical treatment instrument according to claim 3, wherein the first gear is configured to be located inside the housing at all times in a process in which the opening and closing pieces pivot from a maximum closed angle to a maximum opening angle.

5. The medical treatment instrument according to claim 1, wherein the rotatable member has a pair of rotating plate members configured to place the second gear between the pair of rotating plate members, separated from each other, and having disc shapes with the same diameter, and
the power transmission member includes:
a first wire wound around a first rotating plate member of the pair of rotating plate members in a predetermined first direction in a direction of rotation of the rotatable member, and
a second wire wound around a second rotating plate member of the pair of rotating plate members in a second direction opposite to the first direction in the direction of rotation of the rotatable member.

6. The medical treatment instrument according to of claim 1, wherein a rotating shaft of the second gear is parallel to a rotating shaft of the first gear.

7. The medical treatment instrument according to claim 1, wherein the first gear is fixed to a first opening and closing piece of the pair of opening and closing pieces,
a second opening and closing piece of the pair of opening and closing pieces has a third gear with a rotating shaft parallel to a rotating shaft of the first gear, and
the second gear transmits a rotational force to the first gear and the third gear such that the first gear and the third gear rotate in opposite directions.

8. The medical treatment instrument according to claim 1, wherein the first gear is fixed to a first opening and closing piece of the pair of opening and closing pieces, and
the second opening and closing piece of the pair of opening and closing pieces are coupled to the first opening and closing piece via a link such that the pair of opening and closing pieces pivot about the opening and closing shaft section in opposite directions.
